# EUROPÄISCHE PATENTANMELDUNG

(11) **EP 0 800 084 A1**
(43) Veröffentlichungstag der Anmeldung: **08.10.1997**
(21) Anmeldenummer: 97105124.8
(22) Anmeldetag: 26.03.1997
(51) Int. Cl.: G01N 33/569, C12Q 1/70

(54) **Technik zum Nachweis von Infektionen mit dem TBE-Virus und anderen Flaviviren**

(30) Priorität: 02.04.1996 DE 19613253
(71) Anmelder: Immuno GmbH, 69020 Heidelberg (DE)
(72) Erfinder: Kiessig, Stephan, Dr., 69168 Wiesloch (DE); Reichel, Margarete, 69120 Heidelberg (DE)
(74) Vertreter: Kolb, Helga, Dr. Dipl.-Chem.

(57) **Zusammenfassung**

Die Erfindung betrifft ein Verfahren zum Testen einer Probe auf Antigene und/oder Antikörper, die für eine Infektion mit einem Virus aus der Familie der Flaviviridae indikativ sind, umfassend die Schritte (a) zur Verfügung stellen einer Reagenskomponente bestehend aus einem von einem Flavivirus abgeleiteten preM-Protein bzw. einem Derivat davon oder einem Antikörper gegen das preM-Protein bzw. gegen ein Derivat davon, (b) Inkubieren der Probe mit der Reagenskomponente für einen Zeitraum, der die Bildung eines Reaktionsproduktes zwischen der Reagenskomponente und eines gegebenenfalls in der Probe enthaltenen preM-Antigens bzw. Antikörpers gegen preM ermöglicht, und (c) Bestimmen des Reaktionsproduktes, welches für eine Infektion mit einem Virus aus der Familie der Flaviviridae indikativ ist.

## Beschreibung

Die vorliegende Erfindung betrifft Verfahren zum Testen einer Probe auf Antigene und/oder auf Antikörper gegen ein Virus aus der Familie der Flaviviridae bzw. Verfahren zum Feststellen einer Infektion mit einem Flavivirus. Weiters werden entsprechende Sets sowie geeignete Reagenzien zur Durchführung der Verfahren zur Verfügung gestellt.

Flaviviren, die der Familie der Togaviridae zugerechnet werden, zählen zu den lipidumhüllten RNA-Viren und umfassen Viren wie beispielsweise das Gelbfiebervirus, das West-Nil-Virus, Dengue-Viren, das Japanische Enzephalitis-Virus, Hepatitis C-Virus oder TBE-Virus wie beispielsweise das TBE-Virus des westlichen Subtyps (FSME-Virus).

Die Übertragung vieler dieser Viren auf Menschen erfolgt hauptsächlich durch Gliedfüssler, z.B. durch Zecken. Diese Viren führen u.a. auch zu Entzündungen des Gehirns, daher auch die Bezeichnung TBE (= tick borne encephalitis)-Virus.

Definitionsgemäss sind alle Flaviviren serologisch verwandt, wie durch Hämagglutinations-Inhibitionstests gezeigt werden kann. Durch Kreuzneutralisation können diese jedoch in mehrere Serokomplexe unterteilt werden, die Viren umfassen, die miteinander näher verwandt sind als mit Mitgliedern anderer Serokomplexe oder mit ungruppierten Flaviviren. Das FSME Virus beispielsweise ist ein Mitglied des sogenannten "Tick-borne" Serokomplexes, dem ausserdem das Louping-Ill-Virus, Langat-Virus, Omsken hämorrhagisches Fieber-Virus, Kyasanur Forest-Disease-Virus und weitere angehören.

Die Genom-Organisation von Flaviviren ist durch cDNA-Klonierung und Sequenzierung einzelner Flaviviren bestimmt worden (siehe dazu beispielsweise Rice et al., 1985, Science 229, 726-733). Entsprechend diesen Sequenzaufklärungen enthält das Genom von Flaviviren ein einzelnes langes offenes Leseraster von ca. 11 000 Nukleotiden, das für alle Struktur- und Nichtstrukturproteine kodiert. Die Gene für die Strukturproteine sind im 5'-Anteil der RNA lokalisiert und umfassen ca. ein Viertel des Genoms in der Reihenfolge C-prM(M)-E. PrM (auch preM) stellt ein Vorläuferprotein von M dar, das in unreifen Formen von Flaviviren vorhanden ist (Shapiro et al., 1973, Virology 56, 88-94). Seine proteolytische Spaltung führt zur Bildung von M, und stellt ein spätes Ereignis im Verlauf der Virusreifung dar. Zusätzlich zu den Strukturproteinen können mehrere virusspezifische Nichtstruktur-Proteine in mit Flavivirus infizierten Zellen gefunden werden. Weitere Proteine von Flaviviren wurden z.B. von Pletnev et al., 1986, FEBS, 3660, 200, No.2, 317-321, sequenziert und beschrieben.

Eine vor allem in Zentraleuropa relativ häufig auftretende Erkrankung, die durch Flaviviren verursacht wird, ist die Frühsommer-Meningoenzephalitis (FSME), eine Erkrankung des Zentralnervensystems. Der hauptsächliche Erreger dieser Erkrankung ist das FSME-Virus, welches vor allem durch Zeckenstiche auf den Menschen übertragen wird. Neben der FSME sind jedoch zahlreiche andere durch Zecken übertragbare Krankheiten bekannt, beispielsweise die "Central Europe Encephalitis" oder die "Russian Spring Summer Encephalitis". Das FSME-Virus ist in vielen europäischen Ländern endemisch. Die Krankheit ist in einigen Ländern wie Österreich, Tschechien, Slowakei oder Ungarn gut dokumentiert, und jedes Jahr werden mehrere Hundert in Krankenhäusern aufgenommene Infektionen registriert. Diese Krankheit stellt somit ein signifikantes Problem für die öffentliche Gesundheit dar. Durch Impfen, beispielsweise mit einem hochgereinigten Formalin-inaktivierten Ganzvirus-Impfstoff, der eine entsprechende Immunantwort hervorruft, kann die Krankheit effektiv verhindert werden.

Wurde eine Person nicht geimpft und wird diese dann von einem FSME-Virus infiziert, so besteht auch die Möglichkeit einer nachträglichen passiven Immunisierung, beispielsweise durch Verabreichung eines FSME-spezifischen Immunglobulinpräparates, um das Ausbrechen einer Erkrankung zu verhindern.

In ungünstigen Fällen ist es möglich, dass nach unvollständiger Immunisierung oder unterlassener Auffrischungsimpfung bzw. nach zu später Gabe von spezifischen Hyperimmunglobulin trotzdem eine klinisch manifeste Infektion auftritt. Hier ist es mit den bislang zur Verfügung stehenden diagnostischen Mitteln nicht möglich, durch Nachweis der Antikörper zwischen einer Infektion und einer aktiven oder passiven Immunisierung zu unterscheiden.

Neben einer effizienten Verhinderung eines Krankheitsausbruchs, ist für die klinische Praxis aber auch eine frühe Erkennung von Infektionen mit Flaviviren von grosser Bedeutung. Ein Infektionsnachweis durch klinische Diagnose in Verbindung mit serologischen Methoden scheint in vielen Fällen unumgänglich zu sein. Die für die serologische Bestimmung bereits verwendeten Tests basieren vorwiegend auf dem Nachweis von Antikörpern gegen das Hüllglykoprotein gpE mittels ELISA (siehe z.B. U. Klockmann, Vaccine Vol.9, 1991,p.42-46).

Auch die als Referenzmethoden eingesetzten Verfahren, wie Hämagglutinationshemmtest oder Neutralisationstest, können nur Antikörper gegen das Hüllprotein erfassen, da einerseits die Funktion dieser Testsysteme von der Integrität des Virus abhängig ist und andererseits nur Antikörper gegen das Hüllglykoprotein E das Virus neutralisieren können.

Neben diesen klassischen Tests konnten in der Zwischenzeit Verfahren etabliert werden, die beispielsweise einen Nachweis der FSME-spezifischen IgM-oder IgG-Antikörper in Serum und/oder in Rückenmarksflüssigkeit ermöglichen. So sind für den Nachweis des FSME-Virus zwei Testsysteme der Firma Immuno AG am Markt erhältlich, die Enzymimmunassays *Immunozym FSME*® und *Enzyquick FSME*®. Beide ELISA-Testsysteme basieren auf dem Nachweis FSME-spezifischer IgG- oder IgM-Antikörper. Im Falle des *Immunozym FSME*® Testsystems dienen als feste Phase Mikrotiterplatten, die mit hochgereinigtem Formaldehydinaktivierten FSME-Ganzvirus beschichtet sind. Beim Testsystem *Enzyquick FSME*® werden Kämme mit je 8 FSME Ganzvirus-beschichteten Testplättchen eingesetzt, welche in die Reihen einer Mikrotiterplatte eingehängt werden können.

Für die serologische Diagnose wird neben ELISA Tests auch bevorzugt die Methode des Western-Blotting eingesetzt, welche im allgemeinen eine höhere Spezifität aufweist. Deshalb wird auch immer öfters vorgeschlagen, den ELISA für ein Screening zu verwenden und den Western-Blot dann zur Bestätigung der mittels ELISA erhaltenen Resultate heranzuziehen.

Aus dem Stand der Technik sind auch seit langem Immunassays für den Nachweis unterschiedlicher Pathogene unter Verwendung von Blotting-Methoden bekannt. Die grundlegende Technik hierzu ist beispielsweise in WO 81 02 790, "New solid supports for proteins for analytical purposes" beschrieben.

Dort ist die diagnostische Verwendung von Proteinen, die zuvor elektrophoretisch aufgetrennt und anschliessend auf poröse Träger aus Nitrocellulose transferiert worden sind, beschrieben.

Aus EP-627 625, "Immunoassay for differential diagnostics" sind beispielsweise Verfahren zur spezifischen Bestimmung von HIV-1 Antigenen, wie beispielsweise p24, gp41, p41, gp36 und p36 unter Verwendung eines Western-Blotting-Systems bekannt.

Eine Vielzahl weiterer Dokumente beschäftigt sich mit Verbesserungen der Western-Blot-Technik, so beispielsweise WO 88 05 536 ("Multicolour Western Blot" von Interferon Sciences Inc.), gemäss der die Methode durch Verwendung unterschiedlicher Labels, beispielsweise unterschiedlicher Enzyme, selektiver gestaltet werden kann.

Mittels der bisherigen Testsysteme zum Nachweis von Flaviviridae-Viren ist lediglich der Nachweis von Antigenen per se möglich, es ist aber, und dies ist speziell bei unklarer Anamnese von Bedeutung, nicht möglich, beispielsweise zwischen Antikörpern aufgrund einer früheren Impfung oder aufgrund einer Infektion zu unterscheiden. Auch die Sensitivität bzw. die Spezifität der Tests ist häufig sehr gering.

Eine Aufgabe der vorliegenden Erfindung ist es deshalb, Möglichkeiten für die frühe und sichere Erkennung von Infektionen mit einem Virus aus der Familie der Flaviviridae bzw. Verfahren zum Testen auf Antikörper und/oder Antigene eines Virus aus der Familie der Flaviviridae zur Verfügung zu stellen. Eine weitere Aufgabe der vorliegenden Erfindung liegt darin, ein Testsystem ausreichender Sensitivität bzw. Spezifität zur Verfügung zu stellen.

Die Aufgabe wird erfindungsgemäss durch den Gegenstand der Ansprüche gelöst.

Überraschenderweise wurde gefunden, dass der selektive Nachweis einer Infektion mit einem Virus aus der Familie der Flaviviridae über das preM-Protein bzw. über einen Antikörper gegen dieses Protein möglich ist, welches/r als Indikator für eine Infektion mit dem entsprechenden Virus gefunden wurde, und dass dieser Nachweis für eine frühe Erkennung einer Infektionen mit diesem Virus zuverlässig ist. Beispielsweise sind mittels der erfindungsgemässen Methode mindestens 80% der Infektionen mit einem Flavivirus qualitativ erkennbar, negative Ergebnisse werden mit 99,9% Sicherheit angegeben. Mittels des erfindungsgemässen Tests ist es auch erstmals möglich, immunisierte Personen von solchen zu unterscheiden, die mit dem Virus infiziert worden sind.

Das erfindungsgemässe Verfahren zum Testen einer Probe auf Antigene und/oder Antikörper, die für eine Infektion mit einem Virus aus der Familie der Flaviviridae indikativ sind, umfasst die folgenden Schritte
a) zur Verfügung stellen einer Reagenskomponente bestehend aus einem von einem Flavivirus abgeleiteten preM-Protein bzw. einem Derivat davon oder einem Antikörper gegen das preM-Protein bzw. gegen ein Derivat davon
b) Inkubieren der Probe mit der Reagenskomponente für einen Zeitraum, der zur Bildung eines entsprechenden Reaktionsprodukts zwischen der Reagenskomponente und eines gegebenenfalls in der Probe enthaltenen preM-Antigens bzw. eines Antikörpers gegen preM ermöglicht und
c) Bestimmen des Reaktionsprodukts, welches für eine Infektion mit einem Virus aus der Familie der Flaviviridae indikativ ist.

Bei der zu bestimmenden Infektion mit einem Virus aus der Familie der Flaviviridae handelt es sich vorzugsweise um die Bestimmung einer Infektion mit einem TBE-Virus, am meisten bevorzugt um die Bestimmung einer Infektion mit TBE-Virus des westlichen Subtyps (FSME-Virus).

Die isolierte Reagenskomponente ist vorzugsweise durch Auftrennen eines Reagens erhältlich, welches neben dem preM-Protein bzw. Antikörpern gegen das preM-Protein auch andere Antigene, Antikörper bzw. entsprechende Derivate, abgeleitet von einem Virus aus der Familie der Flaviviridae, enthält.

Das Reagens enthält beispielsweise Glykoprotein-, Membranprotein- oder Hüllprotein-Antigene bzw. entsprechende Antikörper gegen diese Proteine. In einer bevorzugten Ausführungsform sind die Komponenten des Reagens von TBE-Virus, am meisten bevorzugt von TBE-Virus des westlichen Subtyps, abgeleitet.

Die Bestandteile des Reagens werden vorzugsweise durch Auftrennen der Bestandteile des Ganzvirus erhalten. Am meisten bevorzugt wird TBE-Virus des westlichen Subtyps aufgetrennt. Es kann sich um ein lebendes Ganzvirus oder um ein inaktives bzw. inaktiviertes Flavivirus handeln.

Die Auftrennung kann hinsichtlich verschiedener physikochemischer Parameter erfolgen, beispielsweise durch Auftrennung nach dem Molekulargewicht, nach der Molekülgrösse oder entsprechend der Ladung der Moleküle.

Somit kann das preM-Protein aus einem Reagens stammen, welches beispielsweise nach Auftrennung eines Ganzvirus zur Verfügung gestellt wird. Die Trennung kann an einer festen Phase oder in flüssiger Phase erfolgen. In jedem Fall erhält man aber ein noch immunologisch aktives Immunreagens, welches mittels herkömmlicher Verfahren gereinigt werden kann. Das preM-Protein kann auch als Derivat zur Verfügung gestellt werden.

Unter einem Derivat des preM-Proteins wird beispielsweise ein Peptid bzw. Polypeptid oder ein Epitop bzw. ein Mimotop (also ein Epitop, welches von einem Antikörper gegen ein Epitop des Originalproteins nicht unterschieden werden kann), welches von preM abgeleitet ist, verstanden. Das Derivat kann auch ein Fragment des preM Proteins eines Flavivirus darstellen oder entsprechend modifiziert sein, beispielsweise durch eine chemische Behandlung. Das Derivat bzw. Fragment kann mittels gängiger chemischer Verfahrensschritte synthetisiert werden. Das preM Protein bzw. ein Derivat davon kann auch gentechnologisch hergestellt werden, beispielsweise durch Einbringen der Nukleinsäuresequenz bzw. entsprechender Fragmente oder Derivate davon in geeignete Vektoren sowie deren Expression in einem geeigneten Wirtsorganismus. Die Herstellungsmethoden für das preM-Protein bzw. das Derivat können im weiteren Verfahrensschritte zur Isolierung bzw. Reinigung der entsprechenden Substanzen umfassen.

Ein geeignetes Derivat des preM-Proteins genügt vorzugsweise dem Auswahlkriterium, dass es mit Antikörpern einer mit Flaviviridae-Virus infizierten bzw. immunisierten Person reagiert.

Der Nachweis kann durch Detektion eines entsprechenden Reaktionsproduktes, beispielsweise in Plasmaproben infizierter bzw. immunisierter Personen, erfolgen.

Mittels gängiger Methoden (siehe z.B. H.Ambrosius: Antiserumgewinnung aus Tieren in: H.Friemel (Hsg.): Immunologische Arbeitsmethoden, Fischer Verlag, Jena 1991, 4.Auflage, S.21-29) können gegen die genannten Proteine bzw. gegen die entsprechenden Derivate Antikörper hergestellt werden. Diese können dann als Bestandteil des Reagens oder als Reagenskomponente zur Durchführung des erfindungsgemässen Verfahrens zur Verfügung gestellt werden. Es kann sich dabei um monoklonale oder polyklonale Antikörper tierischen oder humanen Urpsrungs handeln.

Die entsprechende Reagenskomponente ist vorzugsweise an einem festen Träger immobilisiert. Dies kann beispielsweise nach Auftrennen des entsprechenden Reagens und Überführen auf eine geeignete Membran, beispielsweise auf eine Nitrocellulosemembran, erfolgen. Die isolierte Reagenskomponente kann auch an einem festen Träger gebunden, wie beispielsweise an einer Mikrotiterplatte zur Durchführung eines ELISA, vorliegen. Weitere Reagenskomponenten sind dann vorzugsweise nicht gemeinsam an der Mikrotiterplatte immobilisiert, sondern in entsprechenden Einsätzen, wie Kämmen oder Mikrokügelchen aus Polystyrol bzw. Latex, vorhanden.

Das gereinigte preM-Protein bzw. ein Derivat davon oder ein gereinigter Antikörper gegen das preM-Protein bzw. gegen ein Derivat davon wird erfindungsgemäss auch für die Herstellung eines Tests für den Nachweis einer Infektion eines Patienten mit einem Virus aus der Familie der Flaviviridae verwendet.

Die zu untersuchende Probe ist vorzugsweise eine biologische Flüssigkeit, beispielsweise Blut, Plasma, Serum oder eine andere Körperflüssigkeit.

Die Inkubation der Probe mit der Reagenskomponente erfolgt unter Bedingungen für einen Zeitraum, der die Bildung eines entsprechenden Reaktionsprodukts zwischen der Reagenskomponente und eines gegebenenfalls in der zu untersuchenden Probe vorhandenen preM-Antigens bzw. eines Antikörpers gegen das preM ermöglicht. Beispielsweise wird für einen Zeitraum zwischen 30 Minuten und 24 Stunden und bei einer Temperatur von 4 bis 37°C inkubiert, vorzugsweise wird 60 bis 90 Minuten bei 25°C in physiologischen Puffern, gegebenenfalls unter Zusatz von Detergentien bzw. Kochsalz, inkubiert.

Das zu bestimmende Reaktionsprodukt, welches für eine Infektion mit einem Virus aus der Familie der Flaviviridae indikativ ist, ist das Produkt aus einer entsprechenden Antigen-Antikörper Reaktion. Bei Verwendung einer Reagenskomponente bestehend aus dem preM-Protein, bildet sich ein Antigen-Antikörper-Komplex bestehend aus dem preM-Protein und den in der Probe vorhandenen Antikörpern gegen dieses preM-Protein. Wird anstelle des preM-Proteins ein geeignetes Derivat des preM-Proteins als Reagenskomponente verwendet, so reagiert auch dieses mit in der Probe gegebenenfalls vorhandenen Antikörpern zu einem entsprechenden Antigen-Antikörper-Komplex.

Die Antigen-Antikörper-Reaktion kann auch als Reaktion von in der Probe gegebenenfalls vorhandenen Flavivirus-Antigenen, wie beispielsweise dem preM-Protein, mit einer Reagenskomponente bestehend aus einem Antikörper gegen das preM-Protein bzw. gegen ein Derivat davon, ablaufen.

Die Bestimmung des Reaktionsprodukts, welches sich durch eine Antigen-Antikörper-Reaktion ergibt und welches für eine Infektion mit einem Virus aus der Familie der Flaviviridae indikativ ist, kann mit Hilfe unterschiedlicher, dem Fachmann bekannter Techniken erfolgen.

Vorzugsweise erfolgt die Bestimmung des spezifischen Reaktionsprodukts wie bei üblichen immunologischen Testsystemen oder unter Verwendung einer Blotting-Technik.

Der Grossteil der verwendeten erfindungsgemässen Tests kann sowohl als homogener als auch als heterogener Assay durchgeführt werden. Das immunologische Testsystem kann je nach Art des verwendeten Markers beispielsweise als ein Enzymimmunassay oder ein Radioimmunassay ausgebildet sein.

In der Regel verwenden die Testsysteme die Antiglobulintechnik als nachweisendes Prinzip. Dazu werden beispielsweise ein bzw. mehrere Virusbestandteile, entsprechende Antikörper oder Derivate davon an einer festen Phase immobilisiert. Nach entsprechender Inkubation mit der zu untersuchenden Probe binden Virus-spezifische Antikörper oder Antigene aus dem Probandenserum, -plasma, -liquor oder einer anderen biologischen Flüssigkeit an diese feste Phase. Die Durchführung eines Waschschrittes führt zur Entfernung unspezifischer, nicht gebundener Anteile. Die Konjugation mit einem markierten Antikörper, beispielsweise mit einem markierten IgG-Antikörper, oder einem markierten Antigen bzw. entsprechenden markierten Derivaten davon und im folgenden die eigentliche Substratreaktion sind Voraussetzung für den Nachweis des gebildeten Antigen-Antikörper-Komplexes.

Als Marker kommen die hierfür gängigen Substanzen in Frage, beispielsweise Enzyme, Chromogene, Luminogene, Fluorogene oder radioaktive Nuklide. Die Bezeichnung des Assays richtet sich nach den verwendeten Markern bzw. Labels und wird dementsprechend dann als Enzym-, Lumino-, Fluoro- oder Radioimmunassay bezeichnet.

Alternativ zur beschriebenen Antiglobulintechnik sind auch sogenannte Capture-Techniken möglich, bei denen beispielsweise entweder zunächst alle Antikörper der gewünschten Antikörperklasse unspezifisch mittels eines antischwere-Kette-Antikörpers gebunden werden oder diese Bindung schon, wie bereits beschrieben, spezifisch an das immobilisierte Antigen stattfindet. Der Nachweis ist dann durch die Reaktion der noch freien Antigen-Bindungsstellen mit einem markierten Antigen möglich.

Alle weiteren bekannten Nachweistechniken für Antikörper, Antigene bzw. entsprechende Antigen-Antikörper-Komplexe in Immunassays, wie beispielsweise kompetitive, indirekte oder homogene Immunassays, sind alternativ einsetzbar.

Neben dem beschriebenen Verfahren zum Nachweis einer Infektion mit einem Flavivirus wird auch ein entsprechendes Set zur Durchführung des Verfahrens zur Verfügung gestellt. Dieses Set enthält neben einer Reagenskomponente, welche aus dem preM-Protein eines Flavivirus bzw. einem Derivat davon oder einem Antikörper gegen das preM-Protein bzw. gegen ein Derivat davon besteht, ein Mittel zur Bestimmung des Reaktionsprodukts zwischen der Reagenskomponente und dem in einer Probe des Patienten, beispielsweise einer Blutprobe, gegebenenfalls vorhandenen preM-Antigen bzw. einem Antikörper gegen preM.

Ein Mittel zur Bestimmung des Reaktionsprodukts ist beispielsweise eine Substanz, die als solche zu einem messbaren Signal führt oder die zu dem messbaren Signal nach Ablauf einer entsprechenden Reaktion, beispielsweise nach Ablauf der Reaktion mit einem Enzym, führt. Das erhaltene Signal ist beispielsweise ein photometrisch detektierbares (chromogen oder fluorogen) oder ein in Zusammenhang mit der Radioaktivität einer Substanz bestimmbares Signal.

Die gereinigte Reagenskomponente liegt vorzugsweise in einer lagerfähigen Form vor, beispielsweise in lyophilisierter oder in getrockneter Form. Sie ist vorzugsweise an einem festen Träger immobilisiert und vorzugsweise von TBE-Virus, am meisten bevorzugt von TBE-Virus des westlichen Subtyps, abgeleitet. Das Mittel zur Bestimmung des Reaktionsprodukts ist bevorzugt ein Enzym-markiertes Antigen oder ein entsprechend markierter Antikörper. Bevorzugterweise wird hierzu Peroxidase verwendet.

In dem Set können als weitere Bestandteile auch entsprechende Standards, also Kalibratoren bzw. Kontrollen, zur Verfügung gestellt werden, welche vorzugsweise in einer lagerstabilen Form vorliegen. diese Standards dienen vorzugsweise einer qualitativen Überprüfung des Tests. Dabei werden sowohl negative als auch positive Kontrollen eingesetzt, vorzugsweise kommen spezifische humane polyklonale Antiseren zum Einsatz, die entsprechenden Kontrollen können aber auch preM-Antigen oder entsprechende Antikörper gegen preM enthalten.

Die vorliegende Erfindung stellt im weiteren ein Verfahren zum Testen einer Probe auf Antikörper gegen ein TBE-Virus bereit. Hierfür wird ein Reagens bereitgestellt, welches von TBE-Virus abgeleitete Antigene bzw. Derivate davon enthält, die an einem festen Träger, vorzugsweise an einer Membran, wie z.B. einer Nitrocellulosemembran, immobilisiert und räumlich voneinander getrennt vorliegen. Der feste Träger kann als Platte, Membran, Streifen, Film oder als Perlen zur Verfügung gestellt werden und u.a. auch aus Papier bestehen. Vorzugsweise sind wenigstens zwei Reagenskomponenten auf einem Trägermaterial räumlich voneinander getrennt oder auf mehreren Trägermaterialien immobilisiert. Es ist aber auch möglich, jede Reagenskomponente für sich auf einem eigenen Membranstreifen zur Verfügung zu stellen; die einzelnen Trägerstreifen können auch in einer sogenannten Kammform miteinander verbunden sein.

Das Reagens zur Durchführung des erfindungsgemässen Verfahrens enthält vorzugsweise eine Reagenskomponente, die im wesentlichen aus einem von TBE-Virus abgeleiteten Antigen bzw. einem entsprechenden Derivat davon besteht. Die Reagenskomponente ist bevorzugterweise ein von einem inaktivierten TBE-Virus abgeleitetes Antigen und liegt in gereinigter Form vor.

Das Antigen kann beispielsweise das preM Protein eines TBE-Virus sein. Ein mögliches Derivat davon kann beispielsweise ein chemisch modifiziertes preM-Protein sein oder ein solches, welches aus einem inaktivierten Ganzvirus erhalten werden kann. Wie bereits früher beschrieben, kommt auch ein rekombinant hergestelltes Antigen oder Antigen-Fragment in Frage. Das Reagens enthält aber vorzugsweise mehrere Reagenskomponenten, wobei eine Reagenskomponente aus preM besteht. Weitere Reagenskomponenten können aus Glyko-E, Glyko-E-Dimer, preME, M und/oder C Antigen bzw. entsprechenden Derivaten davon bestehen. Die Reagenskomponenten liegen vorzugsweise in einem bestimmten Verhältnis gemischt vor.

Vorzugsweise liegen die Komponenten in einem physiologischen Verhältnis vor. Insbesondere liegen sie in einem Verhältnis vor, welches jenem entspricht, das bei Proben von mit Flaviviridae-Viren infizierten Personen gefunden wird.

Ist die Reagenskomponente abgeleitet von einem inaktiven bzw. inaktiviertem Flavivirus, so entspricht das Verhältnis der einzelnen Reagenskomponenten in dem erfindungsgemässen Verfahren vorzugsweise dem bei der Auftrennung dieser Viruskomponenten erhalten. Bevorzugterweise ist die Reagenskomponente an preM-Antigen angereichert.

Die zu bestimmende Probe mit den gegebenenfalls vorhandenen Antikörpern wird mit dem Reagens für eine für die entsprechende Immunreaktion ausreichend lange Zeitdauer inkubiert, und die Reaktionsprodukte (Immunkomplexe) werden, vorzugsweise nach Entfernen ungebundener Bestandteile, bestimmt. Die Inkubation wird entsprechend den bereits an früherer Stelle offenbarten Bedingungen durchgeführt. Auch die verwendeten Nachweisverfahren entsprechen den bereits beschriebenen Methoden.

Spezifische Antikörper gegen ein Gemisch aus verschiedenen Proteinen, wie dies beispielsweise bei Verwendung von Ganzviren der Fall ist, lassen sich nach der Auftrennung des Gemisches entsprechend physikochemischer Parameter, wie z.B. Molekulargewicht, isoelektrischer Punkt und weitere, beispielsweise in der SDS-Elektrophorese nach einem Transfer auf Membranen, z.B. auf Nitrocellulosemembranen, nachweisen. Dieser Vorgang, unter dem Begriff Western-Blotting bereits seit langem bekannt, ist auch zur Durchführung des erfindungsgemässen Verfahrens besonders geeignet.

Die vorliegende Erfindung soll durch die folgenden Beispiele näher erläutert werden, ohne diese jedoch darauf zu beschränken.

### 1.) Auftrennen der FSME-Virus Antigene

Formalin-inaktiviertes FSME-Ganzvirus (IMMUNO AG, Österreich) wird in die Antigene entsprechend ihrem Molekulargewicht aufgetrennt. Für diese Auftrennung wurde die Gelpräparation nach Laemmli et al., (Nature 227, 1970, S. 680-685) geringfügig modifiziertgeändert und eine Gelkonzentration von 12,5% unter Zusatz von Glycerin und EDTA verwendet; die Dicke des Gels betrug 1,5 mm.

Die Zusammensetzung des Elektrodenpuffers wurde gemäss der Originalrezeptur von Laemmli verwendet.

Tabelle 1 zeigt die entsprechende Zuordnung einzelner aufgetrennter Banden zu den Strukturproteinen des FSME-Virus. Das preM-Protein konnte als reine Reagenskomponente identifiziert werden (Bandennummer 9).

**Tabelle 1**

| **Banden Nr.** | **MW (kD) +/- 10%** | **Eiweissfärbung** | **Zuckerfärbung** | **FSME- Virus- Protein** |
|---|---|---|---|---|
| 0 | 145 | + | - | Oligo-Glyko E |
| 1 | 137 | + | - | Oligo-Glyko E |
| 2 | 125 | + | - | Oligo-Glyko E |
| 3 | 103 | + | + | Glyko E₂ |
| 4 | 99 | - | - | unbekannt |
| 5 | 71 | - | - | unbekannt |
| 6 | 66 | + | + | preM-Glyko E |
| 7 | 56 | + | + | Glyko E |
| 8 | 40 | - | - | unbekannt |
| 9 | 18 | + | - | preM |
| 10 | 12 | + | - | unbekannt |
| 11 | 11 | - | - | unbekannt |
| 12 | 8,75 | + | - | C |
| 13 | 7,61 | + | - | M |

### 2.) Transfer der Banden auf Membranen

Die entsprechend 1.) aufgetrennten Antigen-Banden wurden unter Verwendung eines Standardpuffers, wie bei Tobwin et al., (PNAS, 76, 1979, p.4350) beschrieben, bestehend aus 25 mmol/l Tris/HCl, pH 8,3, 20%V/V Methanol mittels der Methode des temperierten Tankblots bei 10°C in einer Transferkammer über Nacht auf verschiedene Membranen transferiert.

Als Auswahlkriterien wurde einerseits die untere Nachweisgrenze für einzelne FSME-Virus-Antigene herangezogen, andererseits die Schneidequalität der Membranen. Die Nitrozellulase-Membran schien diesen Anforderungen am besten gerecht zu werden.

### 3.) Blockierung

Die Blockierung erfolgte mit Phosphatpuffer unter Zusatz von 10 %v/v Glycerin als Weichmacher. Der Zusatz von 1%w/v BSA und 1%w/v Saccharose erwies sich für die Erhaltung der weiteren Lagerstabilität als günstig.

Ein Konzentrat ist beispielsweise bei Raumtemperatur für 2 Jahre lagerstabil, in verdünnter Form beträgt die Lagerstabilität bei 4°C etwa 6 Monate.

### 4. Puffer, Kalibratoren + Kontrollen

Der Arbeitspuffer wurde so optimiert, dass sowohl die Rekonstitution der Kontrollen, des Konjugates, die Verdünnung von Proben und Konjugat und somit alle Inkubationsschritte als auch die Waschschritte mit nur einem aus einem 10-fach-Pufferkonzentrat herzustellenden Inkubations-Waschpuffer durchzuführen sind. Folgende Pufferzusammensetzung wurde verwendet (Angabe in der finalen Pufferverdünnung):
- 0.01: mol/l Tris/HCl bei pH 7.5
- 0.3: mol/l NaCl
- 0.1: % v/v Tween 20
- 0.1: % w/v BSA
- 0.001: % w/v Merthiolat
- 0.001: % w/v Phenolrot

Als Kontrollen wurden einerseits lyophilisierte FSME-IgG-negative Humanseren (negativ-Kontrolle), andererseits lyophilisierte FSME-IgG-positive Humanseren unterschiedlicher Konzentrationen ( 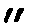 low level 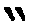 -Kontrolle und 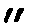 high level 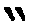 -Kontrolle) eingesetzt. Die Kontrollen wurden in Arbeitspuffer rekonstituiert.

### 5.) Testdurchführung

Die nach 1. aufgetrennten und nach 2. auf Nitrocellulose transferierten Antigene des FSME-Virus wurden mit verdünnten Serum-bzw. Plasmaproben von Patienten auf Streifen dieser Membranen inkubiert. Dabei kommt es zur spezifischen Bindung der in der Probe gegebenenfalls vorhandenen Antikörper an die einzelnen antigenen Banden des aufgetrennten und immobiliserten FSME-Virus.

In einem nachfolgenden Waschschritt wurden nicht gebundene und damit nicht spezifische Bestandteile der Probe entfernt. Das zuvor spezifisch gebundene IgG wurde unter Verwendung Peroxidase-markierter anti-human IgG-Antikörper in der Konjugatreaktion umgesetzt (siehe z.B. Avrameas S., Scand.J.Imunol.8, 1978, p.7-23 oder M.B. Wilson et al., in: Immunofluorescent relating staining techniques, W.Knapp, K.Holubar, G.Wick (eds.), Elsevier, Amsterdam, 1978, p.215-221). In einem weiteren Waschschritt wurde dann ungebundenes Konjugat entfernt.

Im letzten Reaktionsschritt, der Substratreaktion, oxidiert das Konjugat mit seinem Peroxidaseanteil das Chromogen DAB (3'3'-Diaminobenzidin) mit Hilfe des Substrats H₂O₂ zu einer gefärbten, unlöslichen Substanz. Diese Reaktion wird durch das Entfernen des Substrat-Chromogen-Gemisches mittels Waschen mit Wasser gestoppt und die Farbintensität als Mass der Konzentration und der Avidität der jeweiligen spezifischen Antikörper bestimmt. Durch visuellen Vergleich mit den mitgelieferten, dokumentierten Kontrollen können die FSME-spezifischen Antikörper für die einzelnen antigenen Banden beschrieben werden. Der Test ist für eine qualitative Bestimmung geeignet.

### 6.) Testbewertung

Für die Testbewertung wurden folgende Kriterien herangezogen:

### positiv/Infektion:

- Es werden mindestens die Banden: Glyko-E-Dimer,
preME,
Glyko-E und preM
erkannt;
- zusätzliche Banden: M und/oder C.

### positiv/Immunisiert:

- Es werden mindestens die Banden: Glyko-E-Dimer,
preME,
Glyko-E erkannt;

- zusätzliche Banden: M und/oder C.

### negativ:

Es wird keine der oben genannten Bandenkombinationen angezeigt.

### 7. Ergebnisse

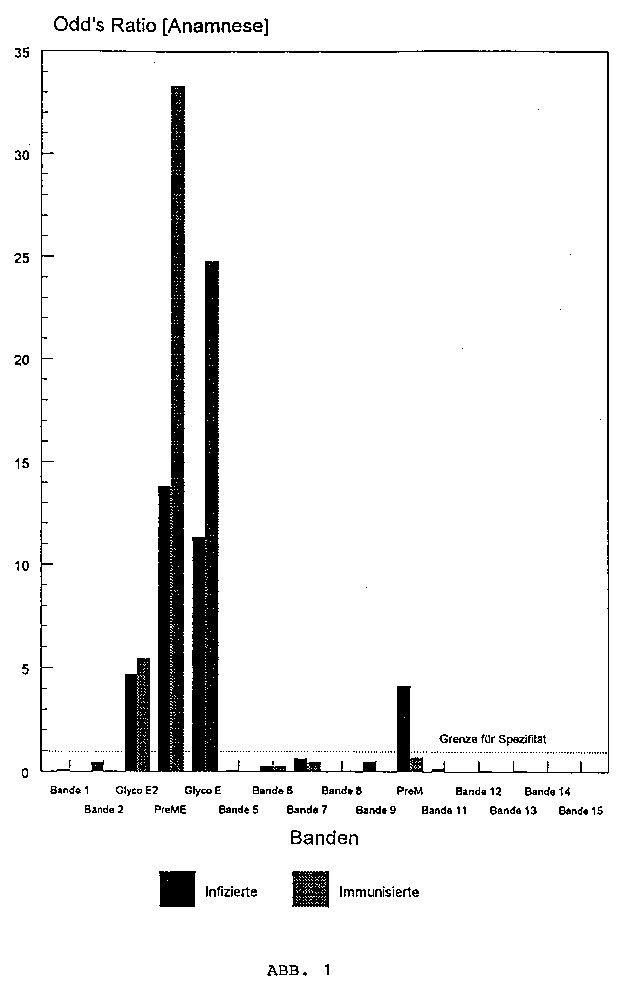

In den nachfolgenden Abbildungen 1 bis 3 sind die Testergebnisse zusammengestellt. Die Abbildungen zeigen dabei im einzelnen:

### Abb. 1:

Die Odd's Ratio in Bezug auf die Anamnese als Mass für die Leistungsfähigkeit des Tests, getrennt nach Immunisierten und Infizierten.
Die Odd's Ratio ist ein gutes Mass für die Richtigkeit einer Vielzahl von Ergebnissen, da die aus der Vierfeldertafel (Anamnese positiv, Anamnese negativ in den Spalten und Test positiv, Test negativ in den Zeilen) zu entnehmenden Zahlen aller richtigen Ergebnisse (richtig positive und richtig negative) durch die aller falschen Ergebnisse (falsch positive und falsch negative) dividiert wird.
Sie ist also um so grösser, je mehr richtig positive und richtig negative Ergebnisse vorliegen.

### Ergebnis:

Es zeigt sich, dass bei Angabe der Odd's Ratio in Bezug auf die Anamnese ausschliesslich die des preM-Proteins eine Unterscheidung Infizierter von Immunisierten möglich macht.

## Patentansprüche

1. Verfahren zum Testen einer Probe auf Antigene und/oder Antikörper, die für eine Infektion mit einem Virus aus der Familie der Flaviviridae indikativ sind, umfassend die folgenden Schritte
a) zur Verfügung stellen einer Reagenskomponente bestehend aus einem von einem Flavivirus abgeleiteten preM-Protein bzw. einem Derivat davon oder einem Antikörper gegen das preM-Protein bzw. gegen ein Derivat davon,
b) Inkubieren der Probe mit der Reagenskomponente für einen Zeitraum, der die Bildung eines Reaktionsproduktes zwischen der Reagenskomponente und eines gegebenenfalls in der Probe enthaltenen preM-Antigens bzw. Antikörpers gegen preM ermöglicht, und
c) Bestimmen des Reaktionsproduktes, welches für eine Infektion mit einem Virus aus der Familie der Flaviviridae indikativ ist.

2. Verfahren gemäss Anspruch 1, dadurch gekennzeichnet, dass das Flavivirus ein TBE-Virus ist.

3. Verfahren gemäss Anspruch 2, dadurch gekennzeichnet, dass das TBE-Virus den westlichen Subtyp darstellt.

4. Verfahren nach einem der Ansprüche 1 bis 3, dadurch gekennzeichnet, dass die Reagenskomponente erhältlich ist durch Auftrennen eines Reagens, welches Antigene, Antikörper oder entsprechende Derivate, abgeleitet von einem Flavivirus, enthält.

5. Verfahren gemäss Anspruch 4, dadurch gekennzeichnet, dass die Reagenskomponente durch Auftrennen nach dem Molekulargewicht, nach Molekulargrösse oder entsprechend seiner Ladung erhältlich ist.

6. Verfahren nach einem der Ansprüche 1 bis 5, dadurch gekennzeichnet, dass die Reagenskomponente an einer festen Phase immobilisiert ist.

7. Verfahren nach einem der Ansprüche 1 bis 6, dadurch gekennzeichnet, dass die Bestimmung des Reaktionsprodukts unter Verwendung enzymmarkierter Immunreaktanten erfolgt.

8. Verwendung des gereinigten preM-Proteins bzw. eines Derivats davon oder eines gereinigten Antikörpers gegen das preM-Protein oder gegen ein Derivat davon für den Nachweis einer Infektion einer Person mit einem Virus aus der Familie der Flaviviridae.

9. Set zum Nachweis einer Infektion mit einem Flavivirus enthaltend
(a) eine Reagenskomponente, welche aus dem preM-Protein eines Flavivirus bzw. einem Derivat davon oder einem Antikörper gegen das preM-Protein bzw. gegen ein Derivat davon besteht, und
(b) Reagentien zur Bestimmung des Reaktionsprodukts von Reagenskomponente und dem in einer Probe des Patienten gegebenenfalls vorhandenen preM Antigen bzw. Antikörper gegen preM.

10. Set gemäss Anspruch 9, dadurch gekennzeichnet, dass die Reagenskomponente an einem festen Träger immobilisiert ist.

11. Set nach einem der Ansprüche 9 oder 10, dadurch gekennzeichnet, dass die Reagenskomponente von TBE-Virus, vorzugsweise des westlichen Subtyps, abgeleitet ist.

12. Set nach einem der Ansprüche 9 bis 11, dadurch gekennzeichnet, dass weiters Standards, vorzugsweise in einer lagerstabilen Form enthalten sind.

13. Verfahren zum Testen einer Probe auf Antikörper gegen ein TBE-Virus, umfassend die folgenden Schritte
a) zur Verfügung stellen eines Reagens, welches von TBE-Virus abgeleitete Antigene bzw. Derivate davon enthält, die an einem festen Träger immobilisiert und räumlich voneinander getrennt vorliegen,
b) Inkubieren der Probe mit dem Reagens für einen Zeitraum, der eine Reaktion der gegebenenfalls in der Probe enthaltenen Antikörper mit dem Reagens ermöglicht, und
c) Bestimmen der Reaktionsprodukte.

14. Verfahren nach Anspruch 13, dadurch gekennzeichnet, dass es sich um einen Western-Blot handelt.

15. Reagens zur Durchführung des Verfahrens gemäss Anspruch 13 enthaltend eine Reagenskomponente, welche aus einem von TBE-Virus abgeleiteten Antigen oder einem entsprechenden Derivat davon besteht.

16. Reagens gemäss Anspruch 15, dadurch gekennzeichnet, dass mehrere Reagenskomponenten enthalten sind, wobei eine Reagenskomponente aus preM besteht.

17. Reagens gemäss Anspruch 15 dadurch gekennzeichnet, dass die Reagenskomponente aus Glyko-E, Glyko-E-Dimer, preME, M und/oder C Antigen bzw. einem Derivat davon besteht.

18. Reagens nach einem der Ansprüche 15 bis 17, dadurch gekennzeichnet, dass die Reagenskomponente(n) ein von einem inaktivierten TBE-Virus abgeleitetes Antigen ist (sind).

19. Reagens nach einem der Ansprüche 15 bis 18, dadurch gekennzeichnet, dass wenigstens zwei Reagenskomponenten auf demselben festen Trägermaterial räumlich voneinander getrennt immobilisiert sind.
